# EUROPEAN PATENT SPECIFICATION

(11) **EP 3 238 672 B1**
(45) Date of publication and mention of the grant of the patent: **17.08.2022**
(21) Application number: 17167450.0
(22) Date of filing: 21.04.2017
(51) Int. Cl.: A61F 5/453

(54) **OUTER BLADDER PROSTHESIS**
ÄUSSERE BLASENPROTHESE
PROTHÈSE VÉSICALE EXTERNE

(30) Priority: 28.04.2016 IT UA20162966
(43) Date of publication of application: 01.11.2017
(73) Proprietor: CASVEN-MED S.r.l., 70124 Bari (IT)
(72) Inventor: CASSISA, Cristoforo, 20090 Segrate (MI) (IT); VENTIMIGLIA, Matteo, deceased (IT)
(74) Representative: Colombo, Stefano Paolo

(56) References cited:
- EP-A1- 0 119 143
- FR-A1- 2 396 541
- GB-A- 2 152 380
- GB-A- 2 293 107
- US-A- 2 864 369
- US-A- 3 406 690

## Description

### BACKGROUND

The present invention relates to the sector of medical aids aimed at solving problems of urinary incontinence. In particular it relates to an outer bladder prosthesis for solving the problem of male incontinence.

### STATE OF THE ART

It is known that the life expectancy of persons, in particular in the developed countries, is constantly increasing.

People are living longer, ageing and having to cope with new pathologies and dysfunctions with which they are forced to live for many years.

Among these, in the field of urology, there exists the problem of urinary urge incontinence. This pathology is due to: a) the natural and paraphysiological dysfunction of the urinary apparatus among the elderly; b) the frequent pathologies affecting the prostate.

Pathologies of the prostate result in ensuing surgical operations and local radiotherapy treatment which are often highly invalidating from both a physical and psychological point of view. For example, there is the problem of continuous dripping of urine and small urinary losses which often give rise to embarrassing hygiene-related situations (dirty and unpleasant-smelling clothing). In turn these situations cause patients to limit autonomously their movements and their social relations.

For these reasons there is a constant increase in the number of urological centres which offer out-patient facilities for the treatment of dysfunctions of the pelvic floor (basically urinary incontinence urge) in an attempt to find a solution to this problem.

At present the aids available consist of: A) pharmacological aids; B) surgical aids; C) "mechanical" aids; D) intravesical urinary catheters with external "gravity" bag; and E) external urinary catheters with a "condom type" fitting system and external "gravity" bag.

GB2293107 discloses a male incontinence bag having an internal diaphragm which divides the bag into upper and lower compartments connected by a hole; the hole being provided with a one-way valve.

EP 0 119 143 A1 discloses a device for the collection of urines of male incontinents. GB 2 152 380 discloses an incontinence appliance for male personal wear.

FR 77 20632 discloses a collection apparatus in particular for people who had a colostomy or a urostomy.

US 3,406,690 discloses a pediatric urine collector.

### SUMMARY OF THE INVENTION

The inventors have noted that the pharmacological aids do not always manage to provide satisfactory results and in any case often have side effects.

The inventors have noted that also the surgical aids do not always provide satisfactory results and in any case are very invasive.

The "mechanical" aids comprise for example absorbent incontinence nappies and bed liners. The inventors have noted that incontinence nappies and bed liners have the problem that they quickly become badsmelling and have a not insignificant cost since a patient must change them several times during the day. The patient moreover is slightly penalized in terms of freedom of movement and may be embarrassed by the fact that the nappy is visible. Moreover the use of nappies is costly since they must be changed more than once during the course of the day. Finally, the use of incontinence nappies and bed liners are a source of serious damage for the environment.

The inventors have also noted that the intravesical urinary catheters with external "gravity" bag are somewhat invasive and pose a high risk of infection. Changing of these catheters may be carried out only by doctors or nurses.

Finally the inventors have also noted that the external urinary catheters with "condom type" fitting system and external "gravity" bag are difficult to position for many elderly patients. In many cases basically it is impossible to keep the catheter in position with the risk of inflammation and abrasion of the skin of the penis.

The inventors have found that the device for the collection of urine of male incontinents disclosed in EP 0 119 143 A1 has some drawbacks. In particular, it consists of two pieces with briefs and a bag to be removably connected to the briefs. This solution has proven to be uncomfortable for the users who are forced to wear the proper briefs and must give up their underwear.

The removable coupling system with hooks and eyelets between the briefs and the bag is generally effective but may be uncomfortable and painful when the person wears his briefs or hangs up the bag to his briefs. In fact it may inadvertently rub the penis with the hooked part of the coupling system.

In addition, the bag is flat and is not comfortable for the user. Finally, since the bag is flat and the hole for the penis is not configured to retain the penis in position, the penis may draw in towards the briefs, thus making the bag ineffective.

The bag disclosed in GB 2 152 380 is cumbersome to carry and especially to wear. It is supported by a belt and belt straps that start from below the groin. The bag cannot be used as a disposable article and must be washed in order to be used again.

The bag disclosed in FR 77 20632 comprises a flat bag and is suitable for patients who had colostomies or urostomies. It is not suitable for patients suffering from urinary incontinence.

The inventors have defined the object of finding an alternative solution to the aforementioned solutions, which does not have the drawbacks of said solutions.

According to the present invention, this object is achieved by a bladder prosthesis comprising a bag having an inner face, an outer face, an upper portion and a lower portion, wherein said upper portion comprises an opening configured to allow the passage through it of the genitals of a male user and an edge configured to close, at least in part, said opening around said genitals of the male user and wherein said lower portion comprises a reservoir for containing the user's urine. The opening has a circular shape of a diameter between about 60 mm and about 90 mm. The outer bladder prosthesis comprises a filter between the upper portion and the lower portion of the bag, wherein said filter allows the passage of fluid from the upper portion to the lower portion but substantially prevents the passage of fluid in the opposite direction. At least a part of the edge of the bag is pleated so as to increase the user's comfort and the bag capacity. The bag has the shape of a tip which is arranged laterally to the lower edge.

The lower portion is shaped as a rectangular trapezium so that a discharge point is situated between the oblique side and the greater base.

Compared to the known solutions, the prosthesis of the invention, advantageously, is much more stable and reliable. This is because it is supported by the genitalis (penis and scrotum) of the user and not only by the penis as in the known solutions. The possibility of inserting the penis and the scrotum into the opening offers more support because the penis of elderly patients often does not provide sufficient virility and is subject to a reduction in its size. On the contrary, the penis and scrotum together are a more reliable support element.

In order to ensure a better grip and, at the same time, ensure a sufficient degree of comfort for the user, the edge of the opening can be elasticized. The elastic edge may be formed by an elastic laminate comprising two layers of non-woven fabric and a plurality of rubber bands disposed parallel to one another. Similar elastic laminates are used, for example, in baby diapers.

According to embodiments, as an alternative or in addition to the elasticized edge, the edge of the opening can be adhesive. Even this arrangement helps to support the bladder prosthesis in a reliable manner.

According to embodiments, as an alternative or in addition to the elasticized edge and/or to the adhesive edge, a belt or the like may be provided in order to support the bag by tying the belt to the user's waist. Even this arrangement helps to support the bladder prosthesis in a reliable manner.

According to embodiments, as an alternative or in addition to the elasticized edge and/or to the adhesive edge and/or to the belt, a front strip of double-sided adhesive tape may be provided for removably fixing the bag to a user's garment, for example, to his briefs or boxer shorts. Even this arrangement helps to support the bladder prosthesis in a reliable manner.

According to the invention, the bag is pleated. On the one hand, this increases the capacity of the bag; on the other hand, the user can wear it comfortably thus significantly reducing the hindrance. Storing and shipping is also favoured in that the folded bag takes up little space.

A filter is provided between the upper portion and the lower portion of the bag. The filter allows the passage of fluid from the upper portion to the lower portion but substantially prevents the passage of fluid in the opposite direction. This is an advantage in that the urine is kept in the lower part of the bag. The user does not run the risk of wetting his genitals with the urine contained in the bag. This makes the bladder prosthesis of the invention suitable for a normal life as well as for sport activities.

Suitable means, such as a discharge tube, may be provided in order to empty the reservoir.

Advantageously, the bag may be shaped in such a way that the lower portion defines a lower point and wherein said discharge tube is arranged at said lower point. In this way the bladder can be completely emptied.

The bag has the shape of a tip and the latter tip is arranged laterally to the lower edge, in order to allow the exit of the tube laterally towards one of the two legs of the pant. In this way, positioning the bag and emptying it is less complicated.

In order to allow a recirculation of air between the outside and the inside of the bag, a ventilation hole may be provided.

### BRIEF DESCRIPTION OF THE FIGURES

The present invention will become clearer from the following description, provided by way of a non-limiting example, to be read with reference to the accompanying drawings, in which:
- Figure 1 is a schematic, inner side, plan view of a bladder prosthesis according to an embodiment of the present invention;
- Figure 2 is a schematic, outer side, plan view of the bladder prosthesis according to Figure 1; and
- Figure 3 is a schematic axonometric view of the bladder prosthesis according to Figure 1.

### DETAILED DESCRIPTION

The bladder prosthesis 10 according to the present invention comprises a storage bag 12 for collecting the urine of a user. The bag 12 comprises an inner face 12a and an outer face 12b. The inner face 12a, when the bladder prosthesis 10 is in use, is directed towards the inside (and at least partially in contact with) the body (pubis) of the user. The outer face 12b, when the bladder prosthesis 10 is in use, is directed outwards, typically towards the underpants of the user.

According to an advantageous embodiment, the bag 12 is at least partially made of a polyvinyl chloride (PVC), for example a medical grade PVC, typically used to manufacture bags for collecting blood or for containing other fluids. Alternatively, the bag 12 could be made of biocompatible material, for example of the type generally used to manufacture bags for colostomies or the like.

The bag 12 has an upper portion 121 and a lower storage portion 122.

Preferably, the upper portion 121a has a tapered form so as to be anatomically adapted for the person using it. For example, the upper portion 121 could have a substantially triangular-shaped form with the vertex truncated and rounded.

The inner face 12a of the upper portion 121 comprises an opening 14. The opening 14 is configured to allow the passage of the penis and the scrotum of the user. The possibility of inserting into the opening 14 the penis and the scrotum offers a greater degree of support since in elderly patients the penis often does not have an adequate virility and is subject to a reduction in its size.

According to embodiments, the opening 14 has a circular form with a diameter of between about 60 mm and 90 mm. Preferably, the opening 14 has a diameter of about 70 mm - 80 mm. In other embodiments, it has an elongated, elliptical or ovoid form. Advantageously, the bag may have different sizes and therefore the opening 14 may have different diameters so as to be adaptable to different users.

Preferably, the opening 14 comprises an elastic edge 16 for allowing, during use, the opening 14 to increase its diameter when the genitals are introduced and to close again around the genitals once they have been introduced. Preferably, the elastic force exerted by the edge 16 is relatively weak, so as not to create pain and discomfort for the user.

According to a preferred embodiment, the edge 16 of the opening 14 comprises an adhesive or a double-sided adhesive tape. Preferably, the adhesive is lined with a removable film. Preferably, the film comprises a tongue (or other similar means) 18 for gripping the film and removing it. Preferably, the film may be removed before inserting the genitals into the opening of the bag. The adhesive ensures a better grip on the skin of the pubis and the perineum and helps keep the bag 12 in position with respect to the genitals.

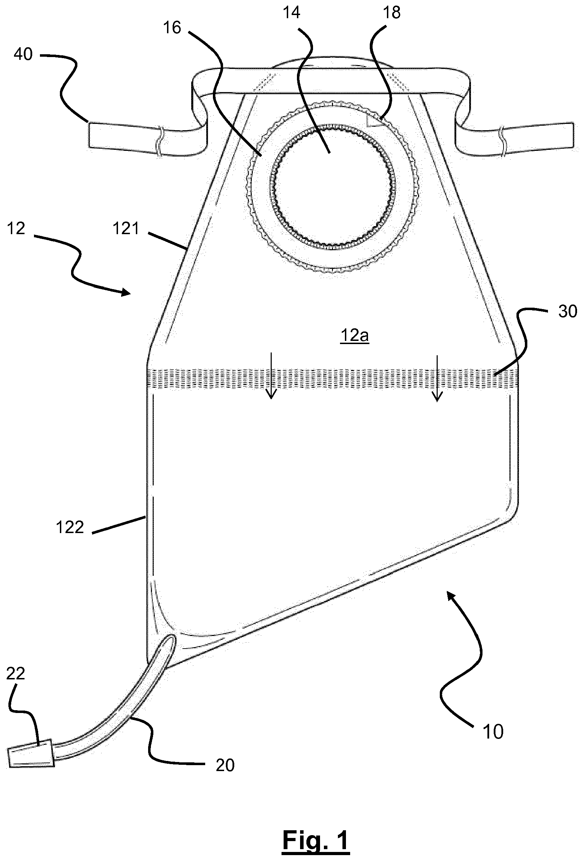

The lower portion 122 of the bag 12 has the function of a reservoir or storage space for containing the user's urine. Preferably, the lower portion 122 has a shape such as to form a discharge point, preventing the presence of other zones where the urine may stagnate. For example, as shown in Figure 1, the lower portion 122 may be shaped as a rectangular trapezium so that the discharge point is situated between the oblique side and the greater base. In other embodiments (not shown), the lower portion 122 has a "pointed" form so that the discharge point is central. The volume of the storage space may about 100-200 ml.

The lower portion 122 comprises a discharge tube 20 for discharging the urine which is collected inside the bag as soon as the user manages to find a bathroom. The tube 20 may have a length of about 150-200 mm.

According to embodiments, the opposite end of the tube 20 is closed by a cap 22 (for example a snap-engaging cap with opening button) or a faucet (for example a three-way drip-feed faucet). In other embodiments the end of the tube 20 is connected to a connection piece. The connection piece serves to connect the discharge tube to a normal urine storage bag of the "gravity" type, for those bed-bound patients who do not like or cannot tolerate conventional intravesical catheterization (unless there are obstructive factors which make the use of a conventional catheter obligatory).

Between the upper portion 121 and the lower portion there is a one-way filter 30 which allows the passage of the urine only from the upper portion 121 to the lower portion 122, but not in the opposite direction. In this way, whatever the movements of the user, the urine is unable to pass from the storage space in the lower portion 122 to the upper portion 121 and therefore the genitals remain dry.

Means may be provided for improving the wearability and the securability.

According to one embodiment, a belt 40 for tying the bag 12 to the user's waist may be provided. The belt 40 may be elastic so as to adapt better to the different dimensions of the users. The belt 40 may be fastened to the upper portion 121 of the bag, preferably above the opening 14. Alternatively, the inner face of the bag may be provided with a through-loop (or several through-loops) through which the belt 40 may be passed. Alternatively, the belt 40 may be fixed by means of one or more buttons.

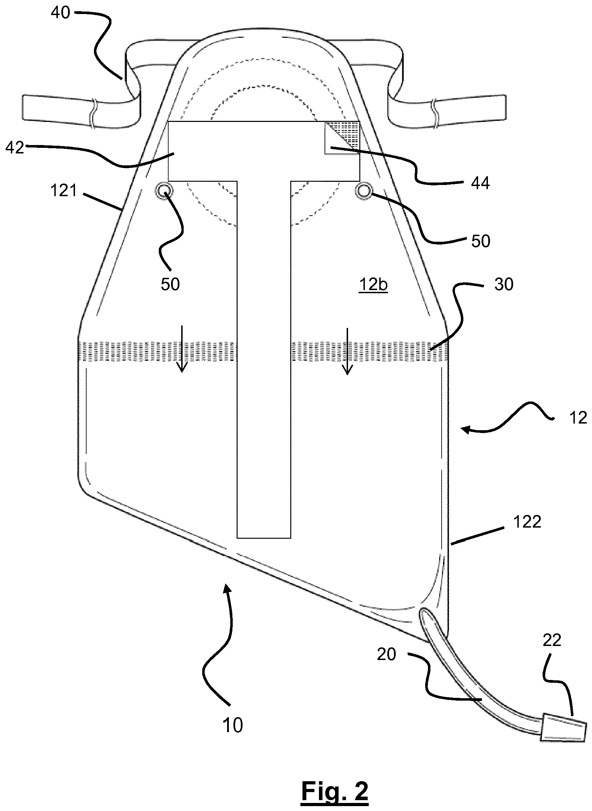

According to one embodiment, a double-sided adhesive tape 42 may be provided on the outer face 12b of the bag 12 so as to fix in a removable manner the bag 12 to the underpants of the user or to another garment. In this way the bladder prosthesis 10 remains stably fixed to the underpants of the user and retained on the user's body. The double-sided adhesive tape 42 could be arranged in a T shape as shown in Figure 2 and could have a tongue 44 for facilitating the removal of the protective film.

According to embodiments, a ventilation hole 50 is provided for allowing recirculation of the air between the outside and inside of the bag 12. Figure 2 shows two ventilation holes 50 on the sides of the opening 14.

According to embodiments, the bag 12 may be formed by joining together and heat-sealing the edges of two shaped sheets (for example made of medical grade PVC).

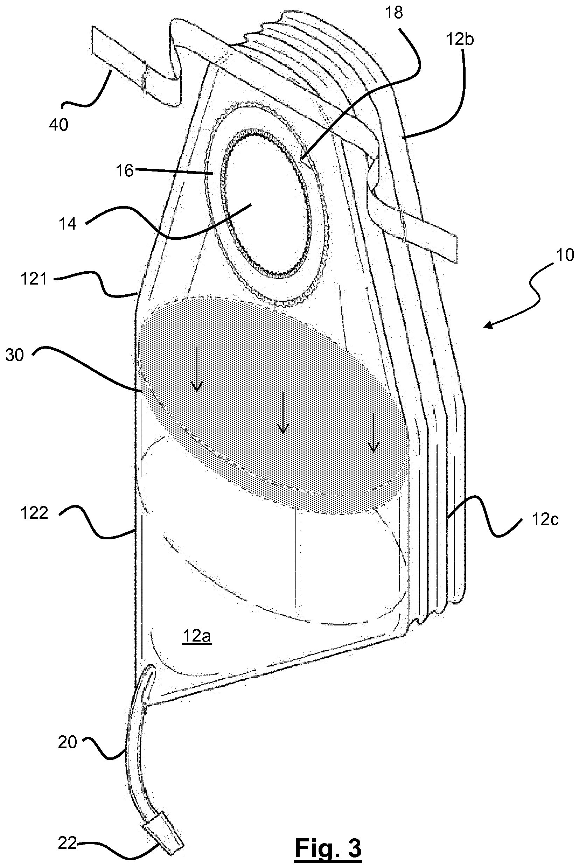

According to other embodiments the bag 12 may comprise a pleated edge 12c (as shown in Figure 3) for allowing a greater internal volume and therefore greater comfort for the user, whose genitals will not be compressed between the two faces of the bag 12. The pleated edges 12c also allow a greater volume of the storage space (lower portion 122 of the bag 12). In any case the pleated edge 12c substantially keeps the bag 12 in a flat form during storage and transportation.

The outer bladder prosthesis 10 according to the invention is preferably for daily disposable use.

The mode of use of the bladder prosthesis 10 according to the present invention may be clearly understood from the above description. The user inserts his penis with the scrotum inside the opening 14. Where necessary, if a double-sided adhesive tape is present along the edge 16 of the opening 14, first he removes the film from the double-sided adhesive tape and then he inserts his genitals. If the belt 40 is present, preferably before inserting his genitals (or if necessary also afterwards) the belt 40 is fastened to the waist so that the bag 12 is supported and cannot fall. Then, if the double-sided adhesive tape 42 is present on the front side 12b of the bag 12, the user removes the associated protective film and, putting on the underpants (of the conventional or disposable mesh type), fixes the bag 12 to the underpants in a removable manner. Alternatively, the bag 12 may be fixed to the underpants also before the user inserts his genitals.

Once in position, if the user suffers from incontinence, the urine passes from the upper portion 121 of the bag 12 to the lower portion 122 where it is collected. The user may empty the bag 12 by means of the tube 20 located at the lower point of the bag 12.

## Claims

1. An outer bladder prosthesis (10) comprising a bag (12) having an inner face (12a), an outer face (12b), an upper portion (121) and a lower portion (122),
i) wherein said upper portion comprises an opening (14) having a circular shape of a diameter between about 60 mm and about 90 mm, said opening (14) being configured to allow the passage through it of the penis and of the scrotum of a male user and having an edge (16) configured to close, at least in part, said opening (14) around the penis and the scrotum of the user,
ii) wherein said lower portion (122) comprises a reservoir for containing the user's urine,
iii) wherein said outer bladder prosthesis (10) comprises a filter (30) between the upper portion (121) and the lower portion (122) of the bag (12), wherein said filter (30) allows the passage of fluid from the upper portion (121) to the lower portion (122) but substantially prevents the passage of fluid in the opposite direction,
iv)wherein at least a part of the edge (12c) of the bag (12) is pleated so as to increase the user's comfort and the bag capacity, and
v) wherein the bag has the shape of a tip which is arranged laterally to the lower edge.

2. The outer bladder prosthesis (10) of claim 1, wherein the lower portion (122) is shaped as a rectangular trapezium so that a discharge point is situated between the oblique side and the greater base.

3. The outer bladder prosthesis (10) of claim 1, wherein the edge (16) of said opening (14) is elastic.

4. The outer bladder prosthesis (10) of claims 1 or 3, wherein the edge (16) of said opening (14) is adhesive.

5. The outer bladder prosthesis (10) of any one of the preceding claims, also comprising a belt (40) for supporting said bag (12) by tying the belt to the user's waist.

6. The outer bladder prosthesis (10) of any one of the preceding claims, also comprising a front strip of double-sided adhesive tape for removably fixing the bag (12) to a user's garment.

7. The outer bladder prosthesis (10) of claim 1, wherein said reservoir comprises means (20, 22) for emptying it.

8. The outer bladder prosthesis (10) of claim 7, wherein said means (20, 22) for emptying the reservoir comprise a discharge tube (20).

9. The outer bladder prosthesis (10) of claim 8, wherein said bag (10) is shaped so that the lower portion (122) defines a lower point and wherein said discharge tube (20) is arranged at said lower point.

10. The outer bladder prosthesis (10) of any one of the preceding claims, also comprising a ventilation hole (50) for allowing recirculation of the air between the outside and inside of the bag (12).

## Patentansprüche

1. Äußere Blasenprothese (10), die eine Beutel (12) umfasst, der eine Innenfläche (12a), eine Außenfläche (12b), einen oberen Abschnitt (121) und einen unteren Abschnitt (122) aufweist,
i) wobei der obere Abschnitt eine Öffnung (14) umfasst, die eine kreisförmige Form mit einem Durchmesser zwischen etwa 60 mm und etwa 90 mm aufweist, wobei die Öffnung (14) dafür konfiguriert ist, den Durchgang des Penis und des Hodensacks eines männlichen Benutzers zu ermöglichen und einen Rand (16) aufweist, der dafür konfiguriert ist, die Öffnung (14) um den Penis und den Hodensack des Benutzers herum zumindest teilweise zu verschließen,
ii) wobei der untere Teil (122) ein Reservoir zur Aufnahme des Urins des Benutzers umfasst,
iii) wobei die äußere Blasenprothese (10) einen Filter (30) zwischen dem oberen Teil (121) und dem unteren Teil (122) des Beutels (12) umfasst, wobei der Filter (30) den Durchgang von Flüssigkeit von dem oberen Abschnitt (121) zu dem unteren Abschnitt (122) ermöglicht, aber den Durchgang von Flüssigkeit in der entgegengesetzten Richtung im Wesentlichen verhindert,
iv) wobei zumindest ein Teil des Randes (12c) des Beutels (12) gefaltet ist, um den Komfort des Benutzers und das Fassungsvermögen des Beutels zu erhöhen, und
v) wobei der Beutel die Form einer Spitze aufweist, die seitlich am unteren Rand angeordnet ist.

2. Äußere Blasenprothese (10) nach Anspruch 1, wobei der untere Teil (122) als rechteckiges Trapez geformt ist, so dass sich eine Auslassstelle zwischen der schrägen Seite und der größeren Basis befindet.

3. Äußere Blasenprothese (10) nach Anspruch 1, wobei der Rand (16) der Öffnung (14) elastisch ist.

4. Äußere Blasenprothese (10) nach Anspruch 1 oder 3, bei der der Rand (16) der Öffnung (14) klebend ist.

5. Äußere Blasenprothese (10) nach einem der vorhergehenden Ansprüche, die darüber hinaus einen Gürtel (40) zum Halten des Beutels (12) durch Binden des Gürtels an der Taille des Benutzers umfasst.

6. Äußere Blasenprothese (10) nach einem der vorhergehenden Ansprüche, die darüber hinaus einen vorderen Streifen aus doppelseitigem Klebeband zur abnehmbaren Befestigung des Beutels (12) an der Kleidung des Benutzers umfasst.

7. Äußere Blasenprothese (10) nach Anspruch 1, wobei das Reservoir Mittel (20, 22) zu seiner Entleerung umfasst.

8. Äußere Blasenprothese (10) nach Anspruch 7, wobei die Mittel (20, 22) zum Entleeren des Reservoirs ein Abflussschlauch (20) umfassen.

9. Äußere Blasenprothese (10) nach Anspruch 8, wobei der Beutel (10) so geformt ist, dass der untere Teil (122) eine untere Stelle definiert und wobei der Abflussschlauch (20) an der unteren Stelle angeordnet ist.

10. Äußere Blasenprothese (10) nach einem der vorhergehenden Ansprüche, die darüber hinaus eine Belüftungsöffnung (50) umfasst, um eine Rückführung der Luft zwischen der Außenseite und der Innenseite des Beutels (12) zu ermöglichen.

## Revendications

1. Prothèse vésicale externe (10) comprenant un sachet (12) ayant une face interne (12a), une face externe (12b), une partie supérieure (121) et une partie inférieure (122),
i) dans laquelle ladite partie supérieure comprend une ouverture (14) ayant une forme circulaire d'un diamètre compris entre environ 60 mm et environ 90 mm, ladite ouverture (14) étant configurée pour permettre le passage à travers celle-ci du pénis et du scrotum d'un utilisateur masculin, et ayant un bord (16) configuré pour fermer, au moins en partie, ladite ouverture (14) autour du pénis et du scrotum de l'utilisateur,
ii) dans laquelle ladite partie inférieure (122) comprend un réservoir destiné à contenir l'usine de l'utilisateur,
iii) dans laquelle ladite prothèse vésicale externe (10) comprend un filtre (30) entre la partie supérieure (121) et la partie inférieure (122) du sachet (12), dans laquelle ledit filtre (30) permet le passage de fluide entre la partie supérieure (121) et la partie inférieure (122), mais empêche sensiblement le passage de fluide dans la direction opposée,
iv) dans laquelle au moins une partie du bord (12c) du sachet (12) est plissée de façon à augmenter le confort de l'utilisateur et la capacité du sachet,
et
v) dans laquelle le sachet possède la forme d'une pointe qui est disposée latéralement par rapport au bord inférieur.

2. Prothèse vésicale externe (10) selon la revendication 1, dans laquelle la partie inférieure (122) est formée comme une trapèze rectangulaire de sorte qu'un point d'évacuation soit situé entre le côté oblique et la base plus large.

3. Prothèse vésicale externe (10) selon la revendication 1, dans laquelle le bord (16) de ladite ouverture (14) est élastique.

4. Prothèse vésicale externe (10) selon la revendication 1 ou 3, dans laquelle le bord (16) de ladite ouverture (14) est adhésif.

5. Prothèse vésicale externe (10) selon l'une quelconque des revendications précédentes, comprenant en outre une ceinture (40) destinée à supporter ledit sachet (12) en serrant la ceinture sur la taille de l'utilisateur.

6. Prothèse vésicale externe (10) selon l'une quelconque des revendications précédentes, comprenant en outre une bande avant de ruban adhésif à double-face destinée à fixer le sachet (12) de manière amovible sur le vêtement d'un utilisateur.

7. Prothèse vésicale externe (10) selon la revendication 1, dans laquelle ledit réservoir comprend un moyen (20, 22) destiné à le vider.

8. Prothèse vésicale externe (10) selon la revendication 7, dans laquelle ledit moyen (20, 22) destiné à vider le réservoir comprend un tube d'évacuation (20).

9. Prothèse vésicale externe (10) selon la revendication 8, dans laquelle ledit sachet (10) est formé de sorte que la partie inférieure (122) définisse un point inférieur, et dans laquelle ledit tube d'évacuation (20) est prévu au niveau dudit point inférieur.

10. Prothèse vésicale externe (10) selon l'une quelconque des revendications précédentes, comprenant en outre un orifice de ventilation (50) destiné à permettre la recirculation de l'air entre l'extérieur et l'intérieur du sachet (12).
